Europäisches Patentamt

European Patent Office

Office européen des brevets

(19)

(11) Veröffentlichungsnummer: **0 221 235 B2**

(12) # NEUE EUROPÄISCHE PATENTSCHRIFT

(45) Veröffentlichungstag der neue Patentschrift:
**22.11.95**

(51) Int. Cl.⁶: **A61N 1/36**

(21) Anmeldenummer: **86107947.3**

(22) Anmeldetag: **11.06.86**

(54) **Vorhofgesteuerter Herzschrittmacher.**

(30) Priorität: **04.10.85 DE 3535543**

(43) Veröffentlichungstag der Anmeldung:
**13.05.87 Patentblatt 87/20**

(45) Bekanntmachung des Hinweises auf die
Patenterteilung:
**31.01.90 Patentblatt 90/05**

(45) Bekanntmachung des Hinweises auf die
Entsheidung über den Einspruch:
**22.11.95 Patentblatt 95/47**

(84) Benannte Vertragsstaaten:
**DE FR GB IT NL SE**

(56) Entgegenhaltungen:
EP-A- 0 064 940      EP-A- 0 081 209
EP-A- 0 108 360      GB-A- 2 073 023
GB-A- 2 076 655      GB-A- 2 116 845
GB-A- 2 142 539

"Physiologische Stimulation des Herzens",
Hrsg. A. Weikl, perimed Fachbuch-Verlags
GmbH, Erlangen, 1984, Seiten 34 bis 36 sowie 49 und 50

"Elektrische Stimulation des Herzens",von B.
Lüderitz, Springer Verlag, Berlin-Heidelberg-
New York, 1980, Seiten 333 und 334

(73) Patentinhaber: **Pacesetter AB**
**Röntgenvägen 2**
**S-171 95 Solna (SE)**

(72) Erfinder: **Hedberg, Sven-Erik, Dipl.-Ing.**
**Odonstigen 5**
**S-196 31 Kungsängen (SE)**
Erfinder: **Lindgren, Anders, Dipl.-Ing.**
**Sportvägen 24**
**S-183 40 Täby (SE)**
Erfinder: **Lekholm, Anders, Dr.**
**Gnejsvägen 4**
**S-161 39 Bromma (SE)**

(74) Vertreter: **Lettström, Richard Wilhelm**
**H. Albihns Patentbyra AB,**
**Box 3137**
**S-103 62 Stockholm (SE)**

EP 0 221 235 B2

# Beschreibung

Bei einem vorhofgesteuerten Herzschrittmacher erfolgt die Erzeugung der ventrikulären Stimulationsimpulse nach einer Verzögerungszeit, die nach dem Auftreten eines Vorhofsignales vergeht.

Ein Herzschrittmacher dieser Art ist in der EP-A-0 064 940 beschrieben. Bei diesem bekannten Herzschrittmacher ist eine in Abhängigkeit vom Auftreten vor Vorhofsignalen vor einer vorgegebenen Grenze, nämlich bei einer Vorhoftachykardie, auf VVI-Betrieb steuerbare Schaltstufe vorhanden.

Bei einem Herzschrittmacher dieser Art kann die Stimulationsfrequenz innerhalb eines relativ weiten Bereiches von z.B. 50 bis 150 pro Minute variieren. Ein vorhofgesteuerter Herzschrittmacher enthält eine Grenzstufe, die die höchste Impulsrate festlegt. Der Herzschrittmacher arbeitet dabei nicht immer völlig sicher, insbesondere dann nicht, wenn ein Vorhofsignal innerhalb bestimmter Grenzen zu einem Stimulationsimpuls auftritt.

Der Erfindung liegt die Aufgabe zugrunde, einen vorhofgesteuerten Herzschrittmacher so auszubilden, daß unabhängig vom zeitlichen Auftreten eines Vorhofsignales in bezug auf einen Stimulationsimpuls immer ein sicherer Betrieb gewährleistet ist, wobei insbesondere ein Schutz gegen externe Interferenz gegeben ist.

Diese Aufgabe ist erfindungsgemäß gelöst durch einen vorhofgesteuerten Herzschrittmacher mit einer in Abhängigkeit vom Auftreten von Vorhofsignalen gesteuerten Schaltstufe, die den Schrittmacher beim Auftreten mindestens eines Vorhofsignales vor einer vorgegebenen Grenze, die sich aus der Differenz zwischen dem kleinsten synchronen Intervall (SSI) bezüglich Ventrikelaktivität und der AV-Verzögerung ergibt, für eine vorbestimmte Anzahl von Stimulationsimpulsen oder natürlichen ventrikulären Aktivitäten auf ventrikel-inhibierten Betrieb (VVI-Betrieb) umschaltet, wobei die Schaltstufe bei ventrikel-inhibiertem Betrieb (VVI-Betrieb) mit einer vorgegebenen niedrigen und hohen Impulsrate den Schrittmacher beim Auftreten eines Vorhofsignales in einem Interferenzintervall nach einem Stimulationsimpuls oder einer natürlichen ventrikulären Aktivität für das nächste Intervall auf VVI-Betrieb mit niedriger Impulsrate umschaltet.

Die Aufgabe kann auch erfindungsgemäß gelöst sein durch einen vorhofgesteuerten Herzschrittmacher mit einer in Abhängigkeit vom Auftreten von Vorhofsignalen gesteuerten Schaltstufe, die den Herzschrittmacher beim Auftreten mindestens eines Vorhofsignales vor einer vorgegebenen Grenze, die sich aus der Differenz zwischen dem kleinsten synchronen Intervall (SSI) bezüglich Ventrikelaktivität und der AV-Verzögerung ergibt, für eine vorbestimmte Zeit auf ventrikel-inhibierten Betrieb (VVI-Betrieb) umschaltet, wobei die Schaltstufe bei ventrikel-inhibiertem Betrieb (VVI-Betrieb) mit einer vorgegebenen niedrigen und hohen Impulsrate den Schrittmacher beim Auftreten eines Vorhofsignales in einem Interferenzintervall nach einem Stimulationsimpuls oder einer natürlichen ventrikulären Aktivität für das nächste Intervall auf VVI-Betrieb mit niedriger Impulsrate umschaltet.

Bei dem erfindungsgemäßen Herzschrittmacher ist auch bei dem Auftreten von Vorhofsignalen innerhalb eines Intervalles, das kleiner als das kleinste Intervall ist, welches durch die höchste zulässige Stimulationsimpulsrate festgelegt ist, ein sicherer Betrieb gewährleistet.

Der Schrittmacher kann dabei dann, wenn ein Vorhofsignal innerhalb des um die AV-Verzögerungszeit verminderten kleinsten programmierten Intervalles zwischen zwei Stimulationsimpulsen auftritt, sowohl für die Erzeugung einer niedrigen als auch einer hohen Impulsrate im VVI-Betrieb umgeschaltet werden, wobei in jedem Fall eine vorbestimmte Anzahl von Stimulationsimpulsen, die z.B. zwischen einem und sechzehn Impulsen liegt, ausgesandt wird. Die im VVI-Betrieb gelieferte Impulsrate liegt demgemäß zwischen der Basisrate und der höchsten synchronen Impulsrate des Schrittmachers.

In dem Fall, in dem ein Vorhofsignal innerhalb des Interferenzintervalles auftritt, kann der Schrittmacher auf eine vorgegebene niedrige Impulsrate umgeschaltet werden, die eine der programmierten Basisraten sein kann. Da diese Betriebsart gegen externe Interferenz schützen soll, sollte die Impulsrate demgemäß wenigstens in der Nähe der Basisrate liegen.

Ein zusätzliches Merkmal des Betriebes mit der höchsten synchronen Impulsrate ist die Möglichkeit, den Herzschrittmacher gegen von ihm erzeugte Tachykardien zu schützen, indem die hohe VVI-Impulsrate so programmiert wird, daß sie etwas niedriger ist als die höchste synchrone Impulsrate. Da eine durch den Herzschrittmacher erzeugte Tachykardie dazu führt, daß der Herzschrittmacher mit der höchsten synchronen Impulsrate stimuliert, hat die vorgegebene Differenz zwischen der höchsten synchronen Impulsrate und der hohen VVI-Impulsrate zur Folge, daß solche durch den Herzschrittmacher verursachten Tachykardien unmöglich sind.

Die Erfindung ist nachfolgend anhand eines in der Zeichnung dargestellten Ausführungsbeispieles näher erläutert.

Es zeigen:

Fig. 1 Impulsverläufe zur Erläuterung des Erfindungsgedankens,

Fig. 2 einen Herzschrittmacher nach der Erfindung, und

Fig. 3 eine Schaltungsvariante des Herzschrittmachers gemäß Figur 2.

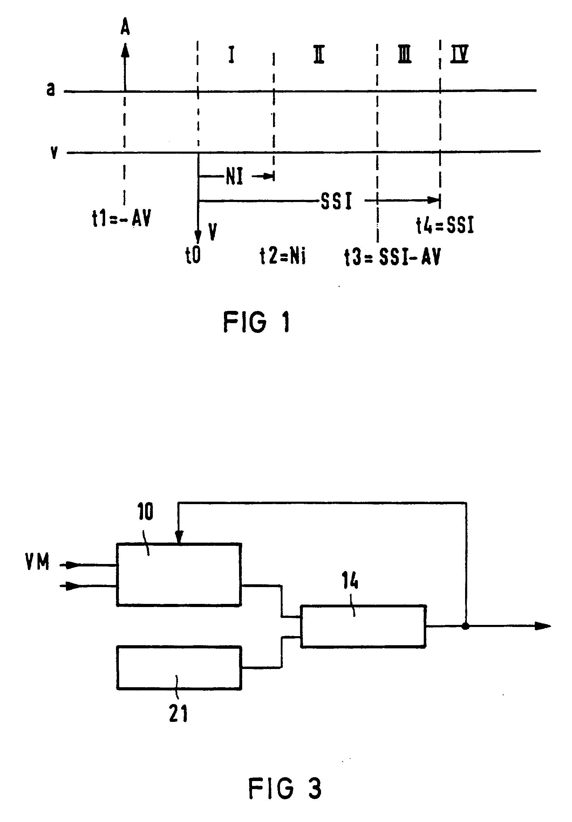

In der Figur 1 ist eine Linie a dargestellt, die die Zeitachse für Vorhofsignale darstellt, sowie eine Linie v, die die Zeitachse für Ventrikelsignale, also Stimulationsimpulse oder natürliche ventrikuläre Aktivitäten, darstellt. Auf der Linie a ist ein Vorhofsignal A und auf der Linie v ein mit der Verzögerungszeit AV darauffolgendes Ventrikelsignal V dargestellt. Der Zeitpunkt $t_4$ legt das Ende des kleinsten Intervalles zwischen zwei Stimulationsimpulsen und damit die höchste synchrone Impulsrate bzw. das kleinste synchrone Intervall SSI fest. Das Intervall zwischen $t_0$ bei V und $t_4$ ist in drei Regionen I, II und III eingeteilt und die Zeit nach dem Zeitpunkt $t_4$ ist mit IV bezeichnet.

Das Intervall zwischen $t_3 = t_4 - AV$ und $t_0$ ist durch einen Zeitpunkt $t_2$ in die beiden Regionen I und II unterteilt. Der Zeitpunkt $t_2$ legt das programmierte Interferenzintervall NI fest.

Der den Kurven gemäß der Zeichnung entsprechend arbeitende Herzschrittmacher besitzt einen Programmspeicher für ventrikel-inhibierten Betrieb (VVI-Betrieb) mit einer vorgegebenen niedrigen und einer vorgegebenen hohen Pulsrate. Die Umschaltung auf VVI-Betrieb mit entsprechender Pulsrate erfolgt durch eine Schaltstufe entsprechend dem zeitlichen Auftreten von Vorhofsignalen in folgender Weise:

Auftreten eines Vorhofsignales in der

Region I Umschaltung auf VVI-Betrieb bei einer niedrigen Impulsrate für mindestens ein Intervall.

Region II Umschaltung auf VVI-Betrieb für eine vorgegebene Anzahl von Stimulationsimpulsen oder natürlichen ventrikulären Aktivitäten oder eine vorgegebene Zeit bei einer vorprogrammierten, hohen Impulsrate.

Regionen III, IV Normaler vorhofgesteuerter Betrieb (DDD-Betrieb).

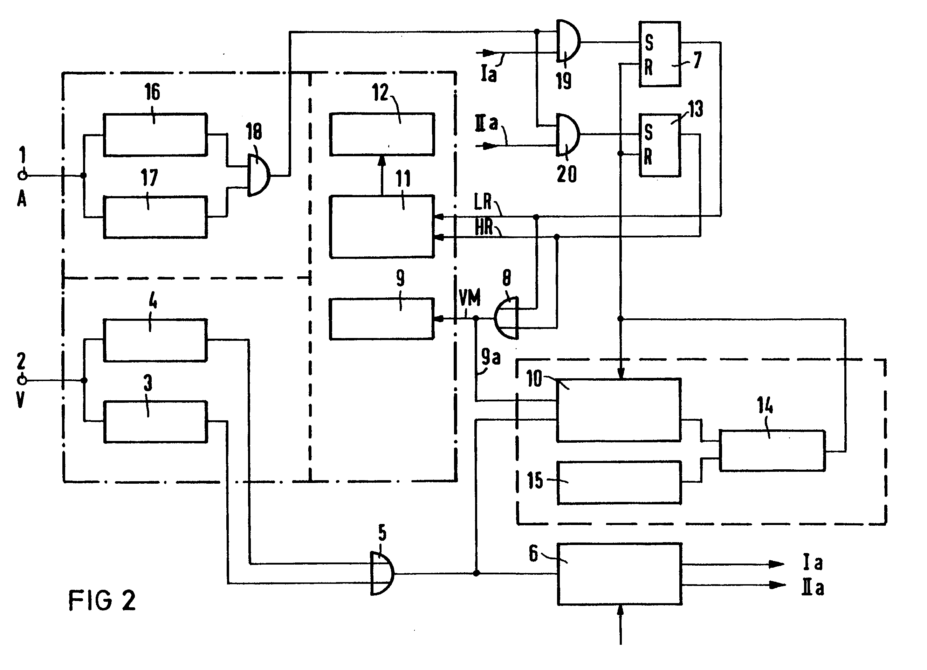

Im Schaltbild gemäß Figur 2 werden die Vorhofsignale einer Klemme 1 zugeführt und die Ventrikelsignale liegen an einer Klemme 2. Pulse einer ventrikulären Ausgangsstufe 3 und eines ventrikulären Detektors 4 gehen über ein ODER-Gatter 5 und starten einen Zeitrechner 6. Der Zeitrechner 6 hat zwei Ausgänge Ia und IIa, welche den Regionen I und II in Figur 1 entsprechen.

Ein Vorhofsignal, das eintrifft, wenn am Ausgang Ia eine logische eins liegt, setzt ein Flip-Flop 7, dessen Ausgangssignal durch ein ODER-Gatter 8 zu einer Mode-Kontroll-Logik 9 weitergeleitet wird, welche zunächst einen Mode-Wechsel vom DDD-Betrieb zu VVI-Betrieb veranlaßt. Dasselbe Signal bewirkt am enable-Eingang eines Rechners 10 eine logische eins, so daß der Zeitrechner 6 zurückgesetzt wird und auf einer logischen "0" bleibt.

Eine Grundfrequenz-Kontrolleinheit 11 empfängt ebenfalls das Ausgangssignal des Flip-Flops 7, wobei ein Grundfrequenzgenerator 12 eine vorbestimmte, niedrige Frequenz erzeugt.

Trifft das Vorhofsignal während der Region II ein, so wird ein Flip-Flop 13 auf Eins geschaltet. Das Ausgangssignal des Flip-Flops 13 geht über das ODER-Gatter 8 zur Mode-Kontroll-Logik 9, die zunächst den VVI-Mode wählt. Der Rechner 10 wird durch ein geeignetes Signal am Eingang 9 aktiviert. Die Grundfrequenz-Kontrolleinheit 11 wird nun von dem Ausgangssignal des Flip-Flops 13 gesteuert, wobei die Grundfrequenz des Herzschrittmachers einen vorbestimmten höheren Wert annimmt.

Wenn am Eingang 9a des Rechners 10 eine logische eins liegt, beginnt der Rechner 10 bei jedem Stimulationsimpuls oder jeder Detektion einer natürlichen Herzaktivität zu rechnen. Es wird also eine vorbestimmte Anzahl von ventrikulären Stimulationsimpulsen und natürlichen Aktivitäten des Herzens vorgegeben, während denen VVI-Betrieb vorliegt.

Ein Komparator 14 vergleicht den Inhalt des Rechners 10 mit einem vorgegebenen Wert n eines Gebers 15. Sind die Inhalte gleich, so gibt der Komparator 14 ein Ausgangssignal, das die Flip-Flops 7, 13 und den Rechner 10 zurücksetzt. Dieses Signal verschwindet, sobald die Flip-Flops 7 und 13 zurückgeschaltet sind. Dann geht auch das Signal des ODER-Gatters 8 zur Mode-Kontroll-Logik 9 auf Null. Dabei bewirkt die Mode-Kontroll-Logik 9 in einer kontrollierten Weise eine Rückkehr auf DDD-Mode.

Die Erfassung der Vorhofsignale erfolgt über einen Detektor 16, dem eine Ausgangsstufe 17 zugeordnet und ein ODER-Gatter 18 nachgeschaltet ist. Das ODER-Gatter 18 steuert die Flip-Flops 7, 13 über zwei UND-Gatter 19, 20.

In der Figur 3 ist eine Variante für den gestrichelt gezeichneten Teil des Herzschrittmachers gemäß Figur 2 dargestellt. Bei dieser Variante ist der Vergleicher 14 mit seinem einen Eingang an einer Zeitreferenzstufe 21 angeschlossen. Wenn am enable-Eingang des Rechners 10 eine logische eins liegt, so wird der Rechner 10 von einem regelmäßigen Taktsignal getaktet. Es wird demgemäß dabei eine vorbestimmte Zeit für den VVI-Betrieb vorgegeben.

## Patentansprüche

1. Vorhofgesteuerter Herzschrittmacher mit einer in Abhängigkeit vom Auftreten von Vorhofsignalen (A) gesteuerten Schaltstufe (Fig. 2), die den Schrittmacher beim Auftreten mindestens eines Vorhofsignales (A) vor einer vorgegebenen Grenze ($t_3$), die sich aus der Differenz zwischen dem kleinsten synchronen Intervall (SSI) bezüglich Ventrikelaktivität und der AV-Verzögerung ergibt, für eine vorbestimmte Anzahl von Stimulationsimpulsen oder natürlichen ventrikulären Aktivitäten auf ventrikel-inhibierten Betrieb (VVI-Betrieb) umschaltet, wobei die Schaltstufe (Fig. 2) bei ventrikel-inhibiertem Betrieb (VVI-Betrieb) mit einer vorgegebenen niedrigen und hohen Impulsrate den Schrittmacher beim Auftreten eines Vorhofsignales in einem Interferenzintervall (I) nach einem Stimulationsimpuls (V) oder einer natürlichen ventrikulären Aktivität für das nächste Intervall auf VVI-Betrieb mit niedriger Impulsrate umschaltet.

2. Vorhofgesteuerter Herzschrittmacher mit einer in Abhängigkeit vom Auftreten von Vorhofsignalen (A) gesteuerten Schaltstufe (Fig. 2), die den Schrittmacher beim Auftreten mindestens eines Vorhofsignales (A) vor einer vorgegebenen Grenze ($t_3$), die sich aus der Differenz zwischen dem kleinsten synchronen Intervall (SSI) bezüglich Ventrikelaktivität und der AV-Verzögerung ergibt, für eine vorbestimmte Zeit auf ventrikel-inhibierten Betrieb (VVI-Betrieb) umschaltet, wobei die Schaltstufe (Fig. 2) bei ventrikel-inhibiertem Betrieb (VVI-Betrieb) mit einer vorgegebenen niedrigen und hohen Impulsrate den Schrittmacher beim Auftreten eines Vorhofsignales in einem Interferenzintervall (I) nach einem Stimulationsimpuls (V) oder einer natürlichen ventrikulären Aktivität für das nächste Intervall auf VVI-Betrieb mit niedriger Impulsrate umschaltet.

## Claims

1. Auricle-controlled heart pace maker having a switching stage (Figure 2), which is controlled as a function of the occurrence of auricle signals (A) and which switches the pace maker over to ventricle-inhibiting mode (VVI-mode) for a predetermined number of stimulation pulses or natural ventricular activities in the event of the occurrence of at least one auricle signal (A) before a predetermined threshold ($t_3$), which is formed from the difference between the smallest synchronous interval (SSI) in respect of ventricular activity and the AV delay, wherein during ventricle-inhibiting mode (VVI-mode) with a predetermined low and high pulse rate, the switching stage (Figure 2), in the event of the occurrence of an auricle signal in an interference interval (I) after one stimulation pulse (V) or a natural ventricular activity, switches the pace maker over to VVI-mode with a low pulse rate for the next interval.

2. Auricle-controlled heart pace maker having a switching stage (Figure 2), which is controlled as a function of the occurrence of auricle signals (A) and which switches the pace maker over to ventricle-inhibiting mode (VVI-mode) for a predetermined period of time in the event of the occurrence of at least one auricle signal (A) before a predetermined threshold ($t_3$), which is formed from the difference between the smallest synchronous interval (SSI) in respect of ventricular activity and the AV delay, wherein during ventricle-inhibiting mode (VVI-mode) with a predetermined low and high pulse rate, the switching stage (Figure 2), in the event of the occurrence of an auricle signal in an interference interval (I) after one stimulation pulse (V) or a natural ventricular activity, switches the pace maker over to VVI-mode with a low pulse rate for the next interval.

## Revendications

1. Stimulateur cardiaque à commande auriculaire, comportant un circuit de commutation (figure 2) qui est commandé en fonction de l'apparition de signaux auriculaires (A) et, lors de l'apparition d'au moins un signal auriculaire (A) avant une limite prédéterminée ($t_3$), qui résulte de la différence entre l'intervalle synchrone le plus faible (SSI) en rapport avec l'activité ventriculaire et le retard AV, commute le stimulateur cardiaque sur le fonctionnement avec inhibition ventriculaire (fonctionnement VVI), pendant un nombre prédéterminé d'impulsions de stimulation ou d'activités ventriculaires naturelles, le circuit de commutation (figure 2) commutant, dans le cas du fonctionnement avec inhibition ventriculaire (fonctionnment VVI) mettant en oeuvre des taux prédéterminées faible et élevé d'impulsions, le stimulateur cardiaque sur le fonctionnement VVI ayant un petit taux d'impulsions lors de l'apparition d'un signal auriculaire pendant un intervalle d'interférence (I) après une impulsion de stimulation (V) ou après une activité ventriculaire naturelle, et ce pendant l'intervalle de temps suivant.

2. Stimulateur cardiaque à commande auriculaire, comportant un circuit de commutation (figure 2) qui est commandé en fonction de l'apparition de signaux auriculaires (A) et, lors de l'apparition d'au moins un signal auriculaire (A) avant une limite prédéterminée ($t_3$), qui résulte de la différence entre l'intervalle synchrone le plus faible (SSI) en rapport avec l'activité ventriculaire et le retard AV, commute le stimulateur cardiaque sur le fonctionnement avec inhibition ventriculaire (fonctionnement VVI), pendant un temps prédéterminé, le circuit de commutation (figure 2) commutant, dans le cas du fonctionnement avec inhibition ventriculaire (fonctionnment VVI) mettant en oeuvre des taux prédéterminées faible et élevé d'impulsions, le stimulateur cardiaque sur le fonctionnement VVI ayant un petit taux d'impulsions lors de l'apparition d'un signal auriculaire pendant un intervalle d'interférence (I) après une impulsion de stimulation (V) ou après une activité ventriculaire naturelle, et ce pendant l'intervalle de temps suivant.

FIG 1

FIG 3

FIG 2